# EUROPEAN PATENT SPECIFICATION

(11) **EP 1 865 074 B1**
(45) Date of publication and mention of the grant of the patent: **20.04.2011**
(21) Application number: 07016364.7
(22) Date of filing: 22.12.1998
(51) Int. Cl.: C12P 19/34, C12Q 1/68

(54) **Direct RT-PCR on oligonucleotide-immobilized PCR microplates**
Direkte RT-PCR auf Oligonukleotid-immobilisierten PCR-Mikroplatten
RT-PCR directe sur microplaques de PCR à oligonucléotides immobilisés

(30) Priority: 22.12.1997 US 68394 P; 16.01.1998 US 71627 P
(43) Date of publication of application: 12.12.2007
(62) Divisional of application: 98964860.5
(73) Proprietor: Hitachi Chemical Co., Ltd., Shinjuku-ku, Tokyo 163-0449 (JP); HITACHI CHEMICAL RESEARCH CENTER, INC., Irvine, CA 92617 (US)
(72) Inventor: Mitsuhashi, Masato, Irvine, CA 92614 (US)
(74) Representative: Vossius & Partner

(56) References cited:
- WO-A-93/09250
- WO-A-93/15228
- WO-A-95/02049
- WO-A-96/31622
- US-A- 5 545 528
- US-A- 5 595 879
- OROSKAR A A ET AL: "DETECTION OF IMMOBILIZED AMPLICONS BY ELISA-LIKE TECHNIQUES" CLINICAL CHEMISTRY, AMERICAN ASSOCIATION FOR CLINICAL CHEMISTRY. WINSTON, US, vol. 42, no. 9, September 1996 (1996-09), pages 1547-1555, XP009010506 ISSN: 0009-9147
- NOLASCO G ET AL: "A METHOD COMBINING IMMUNOCAPTURE AND PCR AMPLIFICATION IN A MICROTITER PLATE FOR THE DETECTION OF PLANT VIRUSES AND SUBVIRAL PATHOGENS" JOURNAL OF VIROLOGICAL METHODS, AMSTERDAM, NL, vol. 45, no. 2, 1993, pages 201-218, XP008006709 ISSN: 0166-0934
- LEVESQUE G ET AL: "REVERSE TRANSCRIPTION AND PCR AMPLIFICATION OF RARE MRNAS IMMOBILIZED ON OLIGO(DT) CELLULOSE" ANALYTICAL BIOCHEMISTRY, ACADEMIC PRESS, SAN DIEGO, CA, US, vol. 213, no. 1, 15 August 1993 (1993-08-15), pages 170-171, XP000616664 ISSN: 0003-2697
- RAINERI I ET AL: "IMPROVED EFFICIENCY FOR SINGLE-SIDED PCR BY CREATING A REUSABLE POOL OF FIRST-STRAND CDNA COUPLED TO A SOLID PHASE" NUCLEIC ACIDS RESEARCH, OXFORD UNIVERSITY PRESS, SURREY, GB, vol. 19, no. 14, 25 July 1991 (1991-07-25), page 4010, XP002023130 ISSN: 0305-1048
- RUPPERT A ET AL: "A FILTRATION METHOD FOR PLASMID ISOLATION USING MICROTITER FILTER PLATES" ANALYTICAL BIOCHEMISTRY, ACADEMIC PRESS, SAN DIEGO, CA, US, vol. 230, no. 1, 1 September 1995 (1995-09-01), pages 130-134, XP000611257 ISSN: 0003-2697
- HAMAGUCHI Y ET AL: "Direct reverse transcription-PCR on oligo(dT)-immobilized polypropylene microplates after capturing total mRNA from crude cell lysates" CLINICAL CHEMISTRY, AMERICAN ASSOCIATION FOR CLINICAL CHEMISTRY. WINSTON, US, vol. 44, no. 11, 1998, pages 2256-2263, XP002208861 ISSN: 0009-9147

## Description

### Background of the Invention

Polymerase chain reaction (PCR) following cDNA synthesis from mRNA (reverse transcription-polymerase chain reaction, RT-PCR) to analyze gene expression of any specific mRNA in cells and tissues has become common technique, because of its better sensitivity and less labor-intensive manipulations than the traditional Northern blot (Kawasaki ES, Wang AM, "Detection of gene expression. In: Erlich, EA. PCR technology", New York: Stockton, 1989;89-97). Furthermore, because recently available recombinant Tth thermostable pofy-merase has activities of both reverse transcriptase and DNA polymerase, both steps can be performed simultaneously in a single tube without changing the buffer system (Myers TW, Gelfand DH, "Reverse transcription and DNA amplification by a Thermus thermophilus DNA polymerase", Biochemistry 1991;30:7661-6). However, it still requires purification of total RNA or mRNA from cells and tissues, which takes additional time-consuming, and labor-intensive step(s).

It has been reported that mRNA is successfully captured by an oligo(dT)immobilized polystyrene (PS) microplate (GENEPLATE^{®}, Hitachi Chemical Co., Japan, and AGCT, Irvine, CA) (Mitsuhashi M, et al., "Gene manipulation on plastic plates", Nature 1992:357:519-20, Miura Y, et al., "Fluorometric determination of total mRNA with oligo(dT) immobilized on microtiter plates", Clin Chem 1996:42:1758-64, Miura Y, et al., "Rapid cytocidal and cytostatic chemosens-rtivity test by measuring total amount of mRNA", Cancer Lett 1997:116:139-44) followed by single- and double-stranded cDNA synthesis on the plate (Tominaga K, et al., "Colorimetric ELISA measurement of specific mRNA on immobilized-origonucleotide-coated microtiter plates by reverse transcription with biotinylated mononucleotides", Clin Chem 1996:42:1750-7). Once double stranded cDNA is formed on a PS microplate of GENEPLATE^{®}, sense stranded cDNA can be removed and used as a template for multiple PCR experiments (Ishikawa T, et al., "Construction of cDNA bank from biopsy specimens for multiple gene analysis of cancer", Clin Chem 1997:43:764-70). Unfortunately, PCR cannot be performed on this PS microplate, because of its heat instability during the denaturing step in PCR cycles. Although heat stable polypropylene (PP) tubes and microplates are primary vessels for PCR, it is difficult to immobilize oligonucleotides onto a PP surface, because of its extremely chamically stable surface characteristics. However, oligo(dT)-immobilized polypropylene plates have recently become available.

As described above, RT-PCR is a very useful technology in various fields including diagnostic molecular pathology (Bortolin S, et al., "Quantitative RT-PCR combined with time-resolved fluorometry for determination of BCR-ABL mRNA", Clin Chem 1996:42:1924-9). However, there are many steps involved in the analysis of RT-PCR; collection of cells, purification of RNA/mRNA, cDNA synthesis, PCR, and quantitation of PCR products. In particular, the purification of intact RNA molecules is the critical first step for the successful RT-PCR, and this requires labor-intensive multiple manipulations to eliminate or inactivate endogenous or contaminated ribonuclease (RNase) in cells and tissues. Although recent PCR technologies allow researchers to continuously monitor the quantity of PCR products with various in-line or off-line verification procedures of amplified PCR products (Morris T, et al., "Rapid reverse transcription-PCR detection of hepatitis C virus RNA In serum by using the TaqMan fluorogenic detection system", J Clin Microbiol 1996:34:p2933-6, Wittwer CT, et at, "The LightCycler: A microvolume multisample fluorimeter with rapid temperature control", BioTechniques 1997:22:171-1811, lack of a simplified RNA preparation system prevents full automation of RT-PCR.

Document WO 93/15228 discloses a method wherein mRNA reverse-transcription is carried out in Sumilon microplates and the antisense cDNA is transferred to a separate container for PCR amplification in solution.

WO 93/09250 and Oroskar et al. (Clinical Chemistry, vol. 42, no. 9, pages 1547-1555), disclose solid-phase PCR amplification in heat-resistant microplates using immobilized oligonucleotide primers.

Nolasco et al. (Journal of Virological Methods, vol. 45, no. 2, pages 201-218) disclose a method combining immunocapture of plant viruses and PCR amplification in a microtiter plate.

US 5 545 528 discloses a method of PCR amplification and detection of nucleic acids in polypropylene microplates.

### Summary of the Invention

In order to simplify the entire process of gene expression analysis, by using oligonucleotide-immobilized polypropylene microplates to which oligonucleotides are securely immobilized and which can be subjected to thermal cycles of PCR (PCR microplates), capturing of mRNA and reverse transcription-polymerase chain reaction (RT-PCR) can be conducted on the same polypropylene plates. Heretofore, microplates were never used for PCR due to insufficient thermal stability, and thus, RT-PCR processes were time-consuming and labor-intensive. In using PCR microplates such as those made of polypropylene, because of their fluorescent characteristics, immobilized oligonucleotide, hybridized mRNA, and synthesized cDNA are quantitated fluorometrically by using nucleic acid stain or enzymatically by producing fluorescence or chemiluminescence. The polypropylene PCR microplates can also capture mRNA from crude cell lysates without purification of RNA or mRNA.

Although hybridized mRNA can be successfully used for one-step RT-PCR with rTth polymerase or two-step RT-PCR with reverse transcription followed by PCR, two-step RT-PCR exhibits higher sensitivity than one-step RT-PCR.

In addition, cDNA synthesized from mRNA captured by immobilized oligonucleotide on the polypropylene PCR microplates can be used more than once, thereby amplifying plural times different or the same portions of the cDNA by using appropriate primers. This muhiple-PCR system, wherein multiple PCRs are synthesized from the cDNA on the polypropylene PCR microplates, is useful in basic research, diagnostics, and drug screening, with potential application to future automation.

Further, conventionally, cell lysate is prepared by vigorous homogenization processes which are not only time-consuming and labor-intensive, but also cause fluctuation of the amount of recovered mRNA. It is also possible, in combination with the polypropylene PCR microplates, by placing target cells on a filter plane evenly and passing a lysis buffer through the cell layer on the filter without disturbing the cells, to drastically simplify the preparation of cell lysate and significantly stabilize the yield of recovered cytosolic RNA. It is very convenient if the microplate and the filter plate are available as a kit for direct RT-PCR. In the above, a lysis buffer, wash buffer, reagents for RT-PCR/PCR, and PBS are commercially available or can readily be prepared so that they need not be included in the kit. However, for convenience, a lysis buffer may be included in the kit for releasing cytosolic mRNA present in the cells when passing through the cell layer on the filter plate. In the above, the lysis buffer comprises a mild detergent for destructing cell membranes but maintaining nuclei to be intact and a reagent for inhibiting RNase activity or inactivating RNase, said lysis buffer having a pH and salt concentration for hybridization.

Because of the above features, the PCR microplates and filter plates can drastically and surprisingly simplify the entire process of RT-PCR from the preparation of cell lysate to measuring specific PCR products with high reliability- Thus, this technology is highly useful in various molecular analyses including basic research, diagnostics, and drug screening, with potential application to future automation.

### Brief Description of the Drawings

Fig. 1 is a graph showing quantities of oligo(dT) immobilized onto oligonucleotide-immobilized polystyrene/polyolefine microplates, indicating that approximately 69% of applied oligonucleotides (10 pmol) were immobilized onto the surface of the microplates.
Fig. 2A is a graph showing mRNA specificity of the PCR microplates, wherein YOYO-1 fluorescent intensity indicates high specificity to mRNA (●) over DNA (■), rRNA (Δ), and tRNA (∇).
Fig. 2B is a graph showing mRNA specificity of the PCR microplates, wherein alkaline phosphatase substrate (ATTOPHOS^{™}) indicates high specificity to mRNA (●) over DNA (■), rRNA (Δ), and tRNA (V).
Fig. 3 is a graph showing reversible hybridization of mRNA on the PCR microplates, wherein YOYO-1 fluorescent intensity indicates sufficient reversibility of mRNA hybridization.
Fig: 4A shows the results of agarose gel electrophoresis showing RT-PCR products from the captured mRNA in crude cell lysates with the expected size of 168 base pairs.
Fig. 4B shows the results of agarose gel electrophoresis showing insignificant false PCR from contaminated genomic DNA in the plates, wherein the band disappeared after being washed with boiling DEPC (diethylpyrocarbonate) water, but not after being washed with 55°C DEPC water.
Fig. 4C shows the results of agarose gel electrophoresis showing PCR conducted with or without reverse transcription.
Fig. 5A is a graph showing hybridized mRNA measured by YOYO-1 at different dilutions of cell suspension. The upper inset shows the results of agarose gel electrophoresis showing hybridized mRNA measured by one-step RT-PCR using rTth polymerase. The lower inset shows the results of agarose gel electrophoresis showing hybridized mRNA measured by two-step RT-PCR using rTth polymerase.
Fig. 5B is a graph showing hybridized mRNA measured by ATTOPHOS™ fluorescence at different dilutions of cell suspension.
Fig. 6A is a graph showing well-to-well variation of the amounts of immobilized oligonucleotides and hybridized rabbit globin mRNA, measured by YOYO-1 fluorescence.
Fig. 6B is a graph showing well-to-weh variation of the amounts of synthesized cDNA from captured rabbit globin mRNA measured by ATTOPHOS™ fluorescence.
Fig. 7 is a graph showing well-to-well variation of the amounts of PCR products inter-assays and intra-assays (upper and lower insets).
Fig. 8 is a graph showing storage stability of the PCR microplates, wherein the amounts of cDNA synthesis were determined by ATTOPHOS™ fluorescence.
Fig. 9 is a graph showing measurement of mRNA and the results of agarose gel electrophoresis of PCR amplification of β*-*actin from various cultured cells which were subjected to lysis on a glass fiber filter to capture mRNA on oligo(dT)-immobilized polypropylene microplates.

### Detailed Description of the Preferred Embodiment

### PCR Microplates

In the present invention, oligonucleotide-immobilized microplates to which oligonucleotides are securely immobilized and which can be subjected to thermal cycles of PCR (PCR microplates) are used, wherein the capture of mRNA and reverse transcription-polymerase chain reaction (RT-PCR) can be conducted on the same plates. The polypropylene PCR microplates can also capture mRNA from crude cell lysates without purification of RNA or mRNA. What makes PCR using polypropylene microplates possible is the use of polypropylene PCR microplates to which oligonucleotides are securely immobilized and which can be subjected to thermal cycles of PCR. For example, conventional oligonucleotide-immobiliaed polystyrene microplates such as GENEPLATE^{®}(AGCT, Irvine, CA) cannot be used for PCR because polystyrene's heat stability is low. PCR microplates usable in the present invention are oligonucleotide-immobilized microplates made of polypropylene. Oligonuleotides immobilized on the polypropylene microplates include, but are not limited to, oligo(dT) and other oligonuleotides specific to mRNA or target RNA. Appropriate sequences can be identified by using HYBsimulator^{™} software (AGCT, Irvine, CA) using hybridization simulation against GenBank primate database (Mitsuhashi M, et al., "Oligonucleotide probe design - a new approach", Nature 1994:367:759-61, Hyndman D, et al., "Software to determine optimal oligonucleotide sequences based on hybridization simulation data", Bio Techniques 1996:20:1090-7). See also US Patent No. 5,556,749, issued September 17, 1996 to Mitsuhashi M, et aL, entitled "Oigo probe designstation: a computerized method for designing optimal DNA probes". The amount of immobilized oligonucleotides can be as high as 10-100 pmol per well (normally 10-30 pmol).

Because of stable surface characteristics of polypropylene, oligonucleotides cannot be immobilized thereon easily. However, recently, some manufacturers produce PCR microplates for molecular biological applications, which can allow researchers to conduct high throughput PCR. In addition, a company such as Hitachi Chemical Research Center (Irvine, CA) can pretreat any commercially available PCR plates to allow oligonucleotides to be immobilized thereonto. Accordingly, this oligonucleotide-immobilized polypropylene plate has recently become available (AGCT, Irvine, CA).

As compared with PS plates or tubes unsuitable for 94°C heat denaturing step in PCR polypropylene, PCR microplates can advantageously be used for PCR. Like widely used PCR microtubes in molecular biological experiments, the polypropylene PCR microplates have low capacity for nonspecific absorption of proteins and DNA/RNA, and resistance to organic chemicals (i.e. phenol/chloroform). These characteristics can be maintained even when oligonucleodites are immobilized thereonto.

Another advantage of oligonucleotide-immobilized polypropylene PCR microplates is the strict specificity to mRNA, but not to rRNA, tRNA or DNA, eliminating the potential problem of false PCR amplification from contaminated genomic DNA, whereas cellulose or beads often contain detectable amounts of rRNA, tRNA and DNA. Furthermore, less variation among wells and plates, excellent stability, and availability of various quality control protocols make this technology very competitive.

Oligonucleotide-immobilized polystyrene microplates have been shown to exhibit a wide variety of applications, which include the capture of total and specific mRNA, ss-cDNA and ds-cDNA synthesis, quantitation of specific mRNA, and sense and antisense mRNA synthesis. oligonucleotide-immobilized polypropylene PCR microplates can also be used for the same purposes as PS microplates. See US Patent No. 5,656,462, issued August 12, 1997 to Keller C, et al., entitled "Method for synthesizing polynucleotide immobilized support".

An interesting feature of the polypropylene PCR microplates is their fluorescent characteristics. Although polypropylene PCR plates are cloudy and not completely transparent compared to PS plates, fluorescence measurement of YOYO^{™}-1 or equivalent dyes exhibited better performance in polypropylene PCR microplates than in PS microplates (Ogura M, Mitsuhashi M, "Screening method for a large quantity of polymerase chain reaction products by measuring YOYO-1 fluorescence on 96-well polypropylene plates", Anal Biochem 1994:218:458-9). See also US Patent No. 5,545,528, issued August 13, 1996 to Mitsuhashi M, et al., entitled "Rapid screening method of gene amplification products in polypropylene plates". This allows for conducting various analysis quite easily. For example, the amounts of both immobilized oligonucleotides, i.e., captured mRNA and synthesized cDNA, can be determined fluorometrically without using radioactive materials.

### Detection of Fluorescence

Because of the fluorescent characteristics of polypropylene, immobilized oligonucleotide, hybridized mRNA, synthesized cDNA, and PCR products are quant-rtated fluorometrically by using nucleic acid stain or enzymatically by producing fluorescence or chemiluminescence, e.g., ATTOPHOS^{™} or LUMIPHOS^{™}. The nucleic acid stains include, but are not limited to, a fluorescent dye selected from the group consisting of 1,1'-(4,4,7,7-tetramethyl-4,7-diazaundecamethylene)-bis-4-(3-methyl-2,3-dihydro-(benzo-1,3-oxazole)-2-methylidenel-quinoliumetraiodide (YOYO^{™}·1), 1,1'-(4,4,7,7-tetramethyl-4,7-diazaundecamethylene)-bis-4-[3-methyl-2,3-dihydro-(benzo-1,3-thiazole)-2-methylidenel-quinoliumetraiodide (TOTO^{™}·1), 1,1'-(4,4,7,7-tetramethyl-4,7-diazaundecamethylene)-bis-4-[3-methyl-2,3-dihydro-(benzo-1,3-thiazole)-2-propenylidene]-quinotiumetraiodid(TOTO^{™}-3), SYBR^{®}-Green 1, 11, and PicoGreen^{®}. See also US Patent No. 5,545,528 described above. Accordingly, this oligonucleotide-immobilized polypropylene, plate has recently become available.

In the following experiments, as a oligonucleotide-immobifized polypropylene PCR microplate, oligo(dT)-immobilized polypropylene microplate GENEPLAT^{®}·PP (Hitachi Chemical Research Center, Irvine, CA) was used. However, oligonucleatide-immobilized polypropylene PCR microplates are not limited to the above, and include any oligonucleotide-immobilized polypropylene microplates to which oligonucleotides are securely immobilized and which can be subjected to thermal cycles of PCR.

### Two-Step RT-PCR or Solid-Phase RT-PCR

There are roughly two ways to perform RT-PCR: one-step RT-PCR and two-step RT-PCR. In one-step RT-PCR, with rTth polymerase or optimal combination of reverse transcriptase and DNA polymerase (Titan^{™}, one tube RT-PCR kit, Boehringer Mannheim, Indianapolis, IN), RT-PCR can be performed on the oligonucleotide-immobilized PCR microplate without changing the buffer. As another one-step RT-PCR, after capturing mRNA by hybridization of mRNA and immobilized oligo(dT), mRNA is removed to a RT-PCR buffer, and RT-PCR can be performed. In contrast, in two-step RT-PCR, after hybridization on oligonucleotide-immobilized polypropylene PCR microplates, captured mRNA is reverse transcribed to cDNA on the same microplates, reactants are removed by aspiration, and PCR is conducted by using heat stable DNA polymerase, e.g., either rTth or Taq polymerase with an appropriate buffer. In the above, PCR is conducted in a thermal cycler with, e.g., 60 cycles of 94°C denaturation for 1 min, 60°C annealing for 1 min followed by 72°C extension for 1 min, by using the same oligonucleotide-immobilized polypropylene PCR microplates as used for hybridization. It is believed that one of ordinary skill in the art expects that one-step or liquid-phase RT-PCR is better than two-step RT-PCR with respect to PCR efficiency. However, when PCR is conducted from synthesized cDNA on the oligonucleotide-immobilized PCR microplates (two-step RT-PCR), RT-PCR can be approximately 10,000-fold more sensitive than conventional one-step RT-PCR, and transcript can be detected from cell lysates containing only 100 cells. This is very surprising because two-step RT-PCR requires inefficient solid phase reverse transcription reaction, whereas one-step RT-PCR is conducted in more efficient liquid phase reaction by first dissociating mRNA from the oligonucleotide-immobilized polypropylene PCR microplates. One explanation may be as follows:
It is believed that primers are used for dimer formation during reverse transcription. More primer dimers are formed in one-step PCR than two-step RT-PCR. Although hybridization efficiency is lower in two-step RT-PCR than one-step RT-PCR, primer dimers formed during initial reverse transcription phase exist throughout PCR in two-step RT-PCR, thereby drastically increasing RT-PCR sensitivity.

### Reamplification by Immobilized cDNA (Multiple-PCR System)

cDNA synthesized from mRNA captured by immobilized oligonucleotide on the oligonucleotide-immobilized PCR microplate can be used more than once, i.e., cDNA on the oligonucleotide-immobilized polypropylene PCR microplate is quite stable. This interesting feature allows reamplification by the same immobilized cDNA to amplify plural times different or the same portions of the cDNA of interest by using appropriate primers. This multiple-PCR system, wherein multiple PCRs are synthesized from the cDNA on the oligonucleotide-immobilized polypropylene PCR microplates, is useful in basic research, diagnostics, and drug screening, with potential application to future automation.

### High Throughput RT-PCR System

Conventionally, cell lysate is prepared by disrupting cells with a lysis buffer to release cytosolic mRNA, followed by centrifugation. Supernatants are used for hybridization. This vigorous homogenization process is not only time-consuming and labor-intensive, but also causes fluctuation of the amount of recovered mRNA. By placing cells on a filter membrane evenly and passing a lysis buffer through the cell layer on the filter membrane without mechanical homoginization of the cells, it is possible to drastically simplify the preparation of cell lysate and significantly stabilize the yield of recovered cytosolic RNA.

Namely, in order to simplify the entire RT-PCR process starting from cells on culture plates, cells are transferred to a filter plate and cells are trapped onto its membrane by vacuum aspiration, positive pressure, or centrifugation. The filter plate is then assembled on top of a origonucleotide-immobilized polypropylene PCR microplate having plural wells, and Lysis buffer is added to each well to mildly destruct cell membranes. After these two-plate sandwiches are centrifuged, cell lysate containing cytosolic mRNA are transferred to the oligonucleotide-immobilized polypropylene PCR microplate for hybridization. After hybridization at room temperature for 1 hour, for example, RT-PCR can be conducted in automated instrument. In the above, a filter or membrane of the filter plate includes, but is not limited to, glass fiber, polypropylene or polyolefine mesh, wool, and other membranes which have a pore size such that target cells can be trapped without any leakage of cells from the membrane, but cytosolic mRNA can pass therethrough. For example, using glass fiber (Grade 934AH, Cambridge Technology, Inc. Watertown, MA) or Whatman GF/F grade glass fiber memberane, most of cultured cells and blood leukocyte can be trapped. In the above, glass fiber plates are preferable. Further, because the filter plate is mounted on the top of a oligonucleotide-immobilized polypropylene PCR microplate, the configuration of the filter plate needs to correspond to that of the oligonucleotide-immobilized polypropylene PCR microplate with respect to, e.g., the number of wells wherein the wells of the filter plate are communicated with the respective wells of the oligonucleotide-immobilized polypropylene PCR microplate when subjected to centrifugation. The maximum capacity of cells per well is normally 10⁴ to 10⁷.

In the above, the cell lysate can be passed through the membrane of the filter plate with the aid of force generated by means of centrifugation, vacuum, or positive pressure. The force necessary to pass the cell lysate through the membrane is easily determined by simple experiments.

In the above, the lysis buffer comprises a detergent for destructing cell membranes, RNase inhibitor for inhibiting RNase activity or deactivating or destroying RNase, and pH control agent and salt for hybridization. In the above, RNase must be active in the lysis buffer. Further, in order to mildly destruct cell membranes so as to prevent contamination of nucleus, the use of a mild detergent is preferable (e.g., NP-40 or TRITON^{™}-X). The above-described lysis buffer is useful and can be used for oligonucleotide-immobilized polypropylene PCR microplates without the use of filter plates.

The protocols of this system include, but are not limited to, the following:
**Step 1: Transfer Cells from Culture Plate to Filter Plate**
   1. Place a filter plate onto a vacuum manifold.
   2. Transfer cells from culture plates to the filter plate by using, e.g., multi-channel pipettes.
   3. Vacuum aspirate the filter plate to trap cells onto membranes.
   4. Wash each well once or twice with, e.g., 50 µl each of PBS (optional).
**Step 2: Transfer Cell Lysate From Filter Plate to oligonucleotide-immobilized polypropylene PCR microplate for Hybridization**
   1. Remove the filter plate from the vacuum manifold and place it on top of the oligonucleotide-immobilized polypropylene PCR microplate.
   2. Add, e.g., 50 µl of lysis buffer (e.g., 10 mM Tris, pH 8.0, 1 mM EDTA, 0.5 M NaCl, 0.5% NP-40, 20 mM vanadyl ribonucleoside complex, RNase-free), and centrifuge at, e.g., 1,500 xg for 10 min.
   3. Incubate the oligonucleotide-immobilized polypropylene PCR microplate at room temperature for 1 hour for hybridization between immobilized oligo(dT) and poly(A) tails of mRNA present in cytosolic fraction of cells.
**Step 3: RT-PCR and Post-PCR Analysis**
   1. Wash each well once or twice with, 50 µl of wash buffer (e.g., 10 mM Tris, pH 8.0, 1 mM EDTA, 0.3 M NaCl, RNase-free).
   2. Start RT-PCR and monitor the amount of PCR products in an automated PCR instrument.

According to the present invention, a rapid, inexpensive, high throughput, and easily automated method for the entire RT-PCR process starting from cells can be realized.

Philadelphia chromosome (Ph¹) found frequently in chronic myelogenous leukemia (CML) is a reciprocal translocation of ABL protooncogene from chromosome 9 to a portion of the BCR gene in chromosome 22 [t(9;22)(q34;q11)] (Wehnert MS, et aL, "A rapid scanning strip for triand dinucleotide short tandem repeats", Nucleic Acids Res 1994:22:1701-4). Specific RT-PCR amplification of BCR-ABL mRNA from peripheral blood cells or bone marrow cells provides a highly sensitive and quantitative methodology for the detection of residual leukemic cells. Because the detection of residual leukemic cells is one of critical indicators for the treatments of CNE, RT-PCR test of BCR-ABL mRNA is widely available in many institutions. However, in many cases, total RNA or mRNA is first purified from cell suspension. Using the present system, once cell lysates are applied to the oligonucleotide-immobilized polypropylene PCR microplates for hybridization, one can proceed with not only direct RT-PCR described here, but also YDYD^{™}-1 quantitation of total amounts of mRNA (Miura Y, et al., Clin Chem 1996:42:1758-64), which may provide additional means of normalization or quality control of tested materials. Because of its simplicity and fluorescent characteristics, oligonucleotide-immobilized polypropylene PCR microplates may be accepted as a platform for various molecular analyses including basic research, diagnostics, and drug screening, with potential application to future automation, especially in combination with filter plates.

### EXAMPLES

The invention will be further explained with reference to Examples shown below. Materials and methods used in the Examples are as follows:
Materials: Oligo(dT)-immbilized oligonucleotide-immobilized PCR microplates (GENEPLATE^{®}-PP, Hitachi Chemical Research Center, Irvine, CA), YOYO^{™}·1 (1,1'-(4,4,7,7-tetramethyl-4,7-diazauhdecamethylene)-bis-4-[3-methyl-2,3-dihydro-(benzo-1,3-oxazole)-2-methytidene]-quinoliumetraiodide, Molecular Probes, Eugene, OR), reagents for PCR (Taq polymerase, EZ rTth RNA-PCR kit) (Perkin Elmar, Foster City, CA), K562 cell line (American Type Culture Collection, Rockville, MD),100 bp DNA ladder, phosphate buffered saline (PBS), vanadyl ribonucleoside complex (YRC), rabbit globin mRNA, cell culture medium and appropriate antibiotics, buffer-saturated phenol (Gibco-BRL, Geithersburg, MD), fetal bovine serum (FBS, HyClone, Logan, UT), biotin-dUTP (Clontech, Palo Alto, CA), ATTOPHOS^{™} (alkaline phosphatase substrate, JBL Scientific, San Luis Obispo, CA), cd4465 DNA (Genome Systems, St. Louis, MO) were purchased from the designated suppliers. RNA preparation reagents for MAGEXTRACTOR^{™} were kindly provided by Toyobo (Osaka, Japan). All other chemicals were purchased from Sigma (St. Louis, MO).
**Cell culture:** K562 cells were grown in RPMI 1640 containing 10% FBS, 500 units/ml penicillin, 500 µg/ml streptomycin, and subcultured twice a week at a ratio of approximately 1:10. Cell viability was assessed by the exclusion of trypan blue, and was always more than 95%.
**Preparation of cell lysate and total RNA**: Cells were washed with PBS twice, and suspended in lysis buffer (10 mmol/L Tris, pH 7.6, 1 mmol/L EDTA, 0.1% NP-40 and 20 mmol/L VRC) on ice for 5 min to release cytosolic mRNA as previously described (Miura Y, et al., Clin Chem 1996:42:1758-64). Samples were then centrifuged at 15,000 xg at 4°C for 5 min, and supernatants were applied to GENEPLATE^{®}-PP for hybridization. In some experiments, cells were suspended in VRC-free lysis buffer, and immediately treated with an equal volume of phenol/chloroform twice to absorb proteins and nucleases. Deproteinated solutions were then subjected to hybridization.
   When a glass fiber filter plate having 96 wells, adapted to be placed on the top of the microplates, was used to recover RNA, the above vigorous homogenization process was entirely omitted. Culture cells were transferred to the glass fiber filter plate by using multi-channel pipettes (described later).
   Total RNA was prepared by automated instrument (MAGEXTRACTOR^{™} MFX-2000, Toyobo, Osaka, Japan). In brief, cell pellets were suspended in kit·supplied caotropic agents, and placed in MAGEXTRACTOR, where RNA was absorbed to the surface of silica felite particles followed by magnet separation. RNA was then automatically eluted to 40 µl of low salt buffer, and was stored at -80°C in a freezer until use. The final RNA was analyzed by agarose gel electrophoresis to confirm 18s and 28s rRNA bands.
**Primer design and synthesis**: Primers for cd4465 (sense: 5'-agtttcggagcggatgaatgc-3', antisense: 5'-ggggcatcagaattttggttga-3'), rabbit globin mRNA (sense: 5'-cgtggagaggatgttcttgg-3', antisense: 5'-aacgatatttggaggtcagcac-3') and ber-abl (sense: 5'-gaccaactcgtgtgtgaaactcca-3', antisense: 5'-aaagtcagatgctactggccgct-3') were designed by HYBsimulator^{™} software (AGCT, Irvine, CA) using hybridization simulation against GenBank primate database (Mitsuhashi M, et al., Nature 1994:367:759-61, Hyndman D, et al., BioTechniques 1996:20:1090-7). In the case of bcr-abl, the sense primer was located at bcr exon 2 and the antisense primer was located at abl exon 2. Primers were synthesized by DNA synthesizer (380B, Applied Biosystem, Foster City), according to the manufacturer's protocol.
One-Step RT-PCR: Template DNA/RNA, 300 µmol/L each of dATP, dGTP, dCTP and dTTP, 1 x EZ buffer, 2 mmol/L Mn(OAc)₂, 0.5 µmol/L each of primer, and 0. 1 µl rTth polymerase were mixed in a final volume of 5-50 µl, and overlayered with one drop of nuclease free mineral oil (Sigma). PCR was conducted in a thermal cycler (MJ Research, Watertown, MA) with 1 cycle of reverse transcription at 60°C for 30 min and 94°C denaturation for 1 min, followed by 40 cycles of 60°C annealing/extension for 1 min and 94°C of denaturation for 1 min. After PCR was completed, PCR products were analyzed by a 2.0 % agarose gel electrophoresis with 0.5 µg/ml ethidium bromide in an electrophoresis chamber. Photographic images were recorded on Polaroid films 1667, Cambridge, MA).
**Two-Step RT-PCR:** After hybridization, captured mRNA was reverse transcribed to cDNA by replacing biotin-dUTP with 10 mmol/L dTTP. Reactants were removed by aspiration, and PCR was conducted by using either rTth or Taq polymerase. For PCR with Taq polymerase, the buffer contained 1x buffer, 1.25 mmol/L MgCl₂, 300 µmol/L each of dATP, dGTP, dCTP and dTTP, 0.5 µmol/L each of primer, and 0.5 µl Taq polymerase in a final volume of 10-50 µl. PCR was conducted in a thermal cycler (MJ Research) with 60 cycles of 94°C denaturation for 1 min, 60°C annealing for 1 min followed by 72°C extension for 1 min.

### Experiment 1: 0uant'rtation of Immobilized Oligonucleotides

GENUNC^{™} PP microplates (Nunc, Naparville, IL) treated at Hitachi Chemical Research Center, Irvine, CA, were obtained from AGCT, Irvine, CA, and oligonucleotides were immobilized thereonto. Oligonucleotide concentrations were determined before and after immobilization as 1.0 OD₂₆₀ unit equals to 30 µg/ml, and the amounts of immobilized oligonucleotides were calculated by subtracting one value from another. In separate experiments, YOYO-1 was diluted in TE (10 mmol/L Tris, pH 8. 0, 1 mmol/L EDTA) in final dilution of 1:1000, and applied to GENEPLATE^{®}-PP microplates. The fluorescence was determined by CYTOFLUOR^{™} 2300 (Millipore, Bedford, MA) with excitation and emission wavelengths of 485 nm (bandwidth 20 nm) and 530 nm (band width 25 nm), respectively, as previously described (Miura Y, et aL, Clin Chem 1996:42:1758-64, Miura Y, et al., Cancer lett 1997:116:139-44).

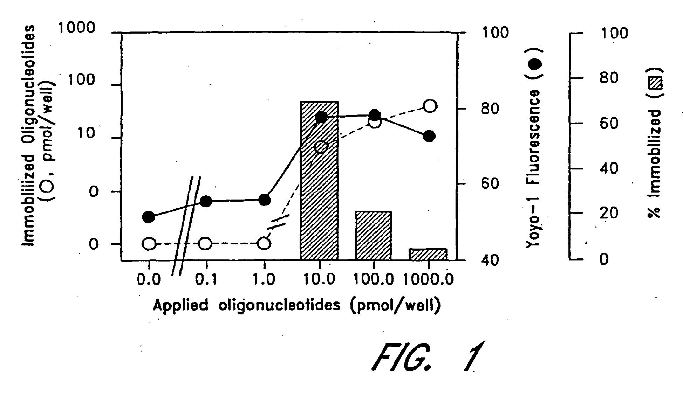

Fig. 1 is a graph showing quantities of ofigo(dT) immobilized onto the oligonucleotide-immobilized PCR microplates. Oligonucleotide concentrations were determined before and after immobilization as 1.0 OD₂₆₀, and the amounts of immobilized oligonucleotides were calculated by subtracting one value from another (○). In parallel experiments, 1:1000 dilution of YOYO-1 was added to each well and its fluorescence was determined (•). Bars indicate the % immobilization from applied oligo(dT)₂₀. Each data point was the mean from triplicate determinations. Although the oligonucleotide-immobilized PCR microplates were opaque and not transparent compared to conventional PS plates, as shown in Fig. 1 (•), the fluorescence of the origonuclectide-immobilized PCR microplates was significantly increased and reached a plateau when more than 10 pmol of oligonucleotides were applied. In further quantitating the actual amounts of immobilized oligonucleotides on the ofgonucleotide-immobilized PCR microplates, as shown in Fig. 1 (○), 21.1 pmol of oligonucleotides were immobilized after 100 pmol of oligonucleotides were applied to each well The amounts of immobilized oligonucleotides were saturated to 10-20 pmol, when more than 10 pmol of oligonucleotides were applied. Approximately 69% of applied oligonucleotides (10 pmol) were immobilized onto the surface of the microplates (Fig. 1, bar graph).

### Experiment 2: mRNA specificity of oligonucleotide-immobitrzed PCR microplates

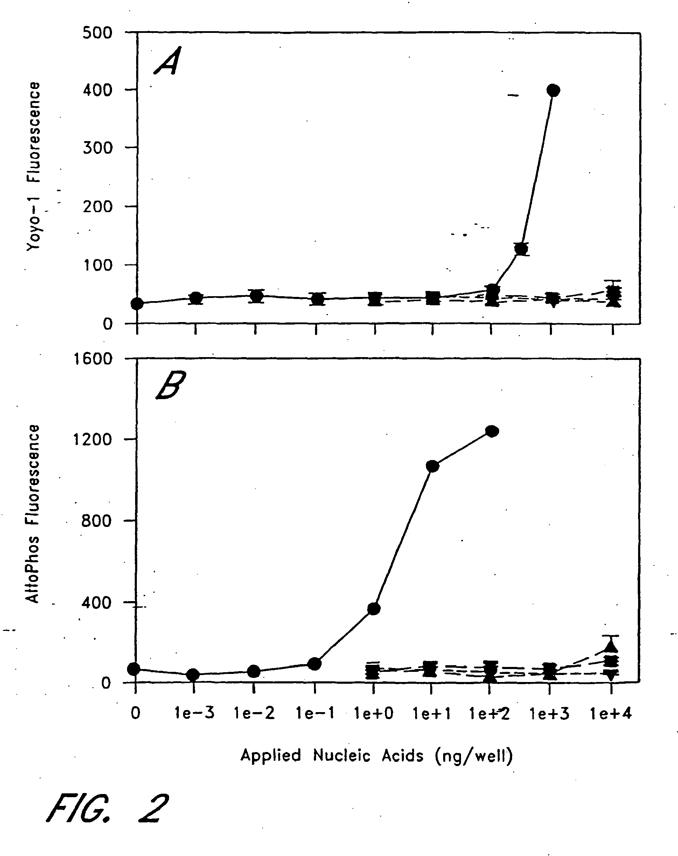

The next series of experiments was conducted to show mRNA specificity. Fig. 2A is a graph showing mRNA specificity of the oligonucleotide-immobilized PCR microplates, wherein YOYO-1 fluorescent intensity indicates high specificity to mRNA (●) over DNA (■), rRNA (Δ), and tRNA (∇). Fig. 2B is a graph showing mRNA specificity of the oligonucteotide-immobitized PCR microplates, wherein substrate ATTOPHOS™ indicates high specificity to mRNA (●) over DNA (■), rRNA (Δ), and tRNA (∇).

In the figures, various concentrations of rabbit globin mRNA (●), DNA (■), rRNA (Δ), tRNA (V) were suspended in 50 µl of hybridization buffer (10 mmol/L Tris, pH 8.0, 1 mmol/L EDTA, 0.5 M NaCl) and applied to the well of the oligonucteotide-immobdized PCR microplates. After hybridization at room temperature for 1 hour, each well was washed once with hybridization buffer. In Fig. 2A, YOYO™-1 was diluted in TE (10 mmol/L Tris, pH 8.0, 1 mmol/L EDTA) in a final dilution of 1:1000, and applied to each well, and the fluorescence was determined by CYTOFLUOR™ 2300. In Fig. 2B, each well was resuspended in 50 µl of cDNA synthesis buffer (50 mmol/L Tris, pH 8.3, containing 75 mmol/L KCI, 3 mmol/L MgC12, 10 mmol/L DTT, 10 mmol/L each of dATP, dGTP, dCTP, 250 µmol/L biotin-dUTP, and 400 U of MMLV reverse transcriptase), and incubated at 37°C for 1 hour. After each well was washed three times with wash buffer (10 mmol/L Tris, pH 7.6, containing 300 mmol/l NaCl and 10 mmol/L Tween 20), 50 µl of wash buffer containing 1:1000 dilution of streptavidin-alkafine phosphatase conjugates and incubated at room temperature for 30 min. After each well was washed three times with wash buffer, 50 µl of substrate (ATTOPHOS^{™}) was added and incubated at room temperature for 20 min. The reaction was terminated by adding an equal volume (50µl) of 100 mmol/L EDTA, and the fluorescence was determined by CYTOFLUOR™ 2300. Each data point was the mean±S.D. from triplicate determinations.

As shown in Fig. 2A, significant YOYO™-1 fluorescence was obtained from the wells where more than 100 ng of mRNA was applied, whereas YOYO™-1 fluorescence was not increased in the wells of rRNA, tRNA and DNA even when as many as 10 µg was applied. The specificity of quantitative cDNA synthesis on the oligonucleotide-immobilized PCR microplates was also tested as described above. As shown in Fig. 2B, significant ATTOPHOS™ fluorescence was obtained from the well where more than 0.1 ng/well of mRNA was applied, but not from the wells of rRNA, tRNA and DNA even when as many as 10 µg was applied.

### Experiment 3: Quantitation of Hybridization

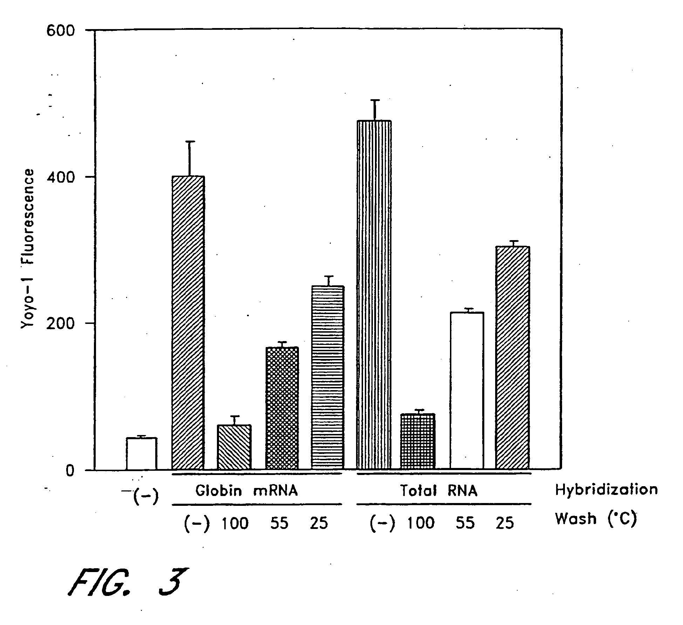

Fig. 3 is a graph showing reversible hybridization of mRNA on the oligonucleotide-immobilized PCR microplates. One µg of rabbit globin mRNA or 20 µg of total liver RNA was suspended in 50 µl of hybridization buffer and applied to the well of the oligonucleotide-immobilized PCR microplates. After hybridization at room temperature for 1 hour, wells were washed three times with DEPC water at different temperatures (25°C, 55°C or boiling). YOYO^{™}·1 was then applied to each well, and the fluorescence was determined by CYTOFLUOR™ 2300 as described above (Miura Y, et al., Clin Chem 1996:42:1758-64). Each data point was the mean+S.D. from triplicate determinations. As shown in Fig. 3, YOYO™-1 fluorescence was reduced to the basal levels by adding boiling DEPC water.

### Experiment 4: Capacity of Hybridization

Moreover, in order to assess the hybridization capacity, various amounts of globin mRNA, total liver RNA or cell lysates were applied to the oligonucleotide-immobilized PCR microplates for hybridization. Hybridized mRNA was then recovered from the plates by adding boiling water, and a buffer (concentration was adjusted) was applied to fresh oligonucleotide-immobitized PCR microplates for the second hybridization. In parallel experiments, known concentrations of globin mRNA were also applied as a control. The quantitative cDNA synthesis described below was then conducted, and the amount of mRNA in the solutions was determined based on the values of standard globin mRNA. The amounts of cDNA synthesis were quantitated according to the protocol published by Tominaga et aL (Clin Chem 1996:42:1750-7) with minor modification. In brief, the mRNA-hybridized ofrgonucleotide-immobifized PCR microplate was resuspended in 50 µl of cDNA synthesis buffer (50 mmol/L Tris, pH 8.3, containing 75 mmol/L KCI, 3 mmol/L MgCl₂, 10 mmol/L DTT, 10 mmol/L each of dATP, DGTP, dCTP, 250 µmol/L biotin-dUTP, and 400 U of MMLV reverse transcriptase), and incubated at 37°C for 1 hour. After each well was washed three times with wash buffer (10 mmol/L Tris, pH 7.6, containing 300 mmol/L NaCl and 10 mmol/L Tween 20), 50 µl of wash buffer containing 1:1000 dilution of streptavidin-alkafine phosphatase conjugates was added and incubated at room temperature for 30 min. After each well was washed three times with wash buffer, 50 µl of substrate (ATTOPHOS^{™}, 1x concentration) was added and incubated at room temperature for 20 mm. The reaction was terminated by adding an equal volume (50 µl) of 100 mmol/L EDTA, and fluorescence was determined by CYTOFLUOR^{™} 2300 (Millipore) with excitation and emission wavelengths of 485 nm (bandwidth 20 nm) and 560 nm (band width 25 nm), respectively.

As shown in Table 1, approximately 50-65% of applied globin mRNA was hybridized to the plates. Applied globin mRNA did not saturate the plates even when 500 ng was used; 500 ng of globin mRNA equals approximately 1-2 pmol compared to 21 pmol of immobilized oligonucleotides. Moreover, approximately 34-48% of total RNA or cell lysates were captured by the plates when mRNA concentration was low, whereas high concentrations decreased capture efficiency, probably because of inefficient hybridization due to high viscosity.

**Table I**

| Applied | Amounts of total mRNA | Captured mRNA (means ± S.D., n-3) | %Capture |
|---|---|---|---|
| globin mRNA | | | |
| 500 ng | 500 ng | 326.7±47.3 ng | 65.3% |
| 50 ng | 50 ng | 32.0±5.6 ng | 64% |
| 5 ng | 5 ng | 2.5±0.3 ng | 50% |

| liver RNA | | | |
|---|---|---|---|
| 50 µg | 583 ng | 45.6±14.2 ng | 7.8% |
| 5 µg | 58.3 ng | 14.8±5.2 ng | 25.4% |
| 0.5 µg | 5.83 ng | 2.8±0.3 ng | 48.0% |

| K562 cells | | | |
|---|---|---|---|
| 10₅ cells | 24.3 ng | 4.2±0.9 ng | 17.2% |
| 10₄ cells | 2.43 ng | 0.83±0.3 ng | 34.1% |

As shown above, the plates are not saturated even when as much as 500 ng of mRNA is applied, which also represents approximately 500 µg of total RNA or 10⁷ cells per small surface area of 96 well plates. This is more than enough for the majority of experiments.

### Experiment 5: RT-PCR in oliqonucleotide-immobilized PCR microplates

Human K562 leukemic cells, which express the b3a2 transcript from the Ph¹ translocation, were lysed with lysis buffer followed by centrifugation to remove cell debris and nuclear DNA. The supernatant containing cytosolic mRNA was then applied to the oligonucleotide-immobitized PCR microplates for hybridization. After 1 hour of hybridization at room temperature, unbound materials were removed by washing with hybridization buffer twice, and RT-PCR was started in the same wells.

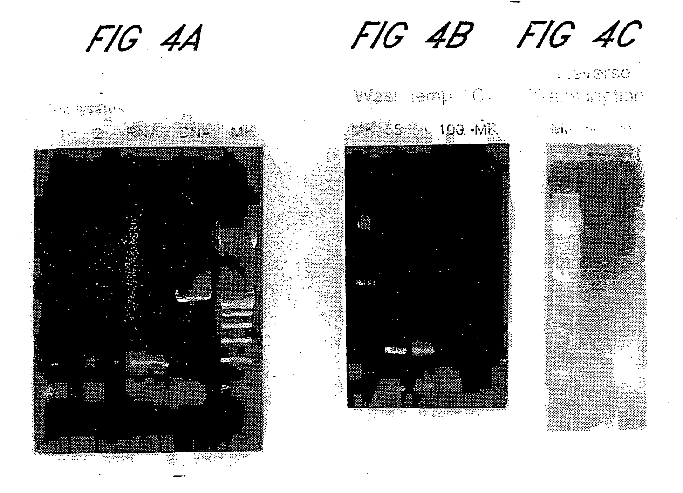

That is, in Fig. 4A, 10⁶ K562 cells were suspended in lysis buffer (10 mmol/L Tris, pH 7.6, 1 mmol/L EDTA, 0.1 % NP-40 and 20 mmol/L VRC) on ice for 5 min to release cytosolic mRNA. Samples were then centrifuged at 15,000 xg at 4°C for 5 min, and supernatants were applied to the origonucleotide-immobitized PCR microplates for hybridization (lane 1). In lane 2, cells were suspended in VRC-free lysis buffer, and immediately treated with an equal volume of phenol/chloroform twice to absorb proteins/nucleases. Deproteinated solutions were then subjected to hybridization. In lane 3, total RNA was prepared by automated instrument as described in the Methods. After hybridization, RT-PCR was conducted in a thermal cycler with 1 cycle of reverse transcription at 60°C for 30 min and 94°C denaturation for 1 min, followed by 40 cycles of 60°C annealing/extension for 1 min and 94°C of denaturation for 1 min, as described in the Methods. Lane 4 was a control cd4465 DNA.

As shown in Fig. 4A (lane 1), BCR-ABL transcript was successfully amplified from the captured mRNA in crude cell lysates with the expected size of 16B base pairs. The size of PCR products was identical to that of PCR products from purified total RNA in the same cells (Fig. 4A, lane 3). Phenol/chloroform treated cell lysates exhibited thicker PCR products than VRC-containing cell lysates (Fig. 4A, lane 2).

In order to analyze the false PCR from contaminated genomic DNA in the plates; mRNA was removed by 55°C or boiling DEPC water and one-step RT-PCR was conducted. That is, in Fig. 4B, after total RNA was hybridized, wells were washed with 55°C DEPC water or boiling DEPC water 3 times, and one-step RT-PCR was conducted. As shown in Fig. 4B, PCR products of BCR-ABL transcript when washed with disappeared when wells were washed with boiling water, but not when washed with 55°C water. These results were comparable to that of Fig. 3.

In separate experiments, PCR was conducted with or without reverse transcription. That is, in Fig. 4C, after total RNA was hybridized, cDNA was synthesized in one tube (+) and one tube was left without reverse transcription (-). PCR was then conducted with Taq polymerase in the presence of 1.25 mM MgCl₂ with 60 cycles of 94°C denaturation for 1 min, 60°C annealing for 1 min followed by 72°C extension for 1 min. PCR products were separated by 2.0% agarose gel electrophoresis followed by staining with ethidium bromide. Mk indicates a 100 bp ladder. As shown in Fig. 4C, PCR products of BCR-ABL transcript were not amplified from the wells of negative reverse transcription even under low stringent conditions with a higher Mg concentration, whereas significant amounts of PCR products were obtained from the wells of positive reverse transcription.

In view of the foregoing, an advantage of the oligonucleotide-immobilized PCR microplates is the strict specificity to mRNA, but not to rRNA, tRNA or DNA (Figs. 2A, 2B, 4A, 4B, 4C), eliminating the potential problem of false PCR amplification from contaminated genomic DNA, whereas cellulose or beads often contain detectable amounts of rRNA, tRNA and DNA.

### Experiment 6: Two-Step RT-PCR

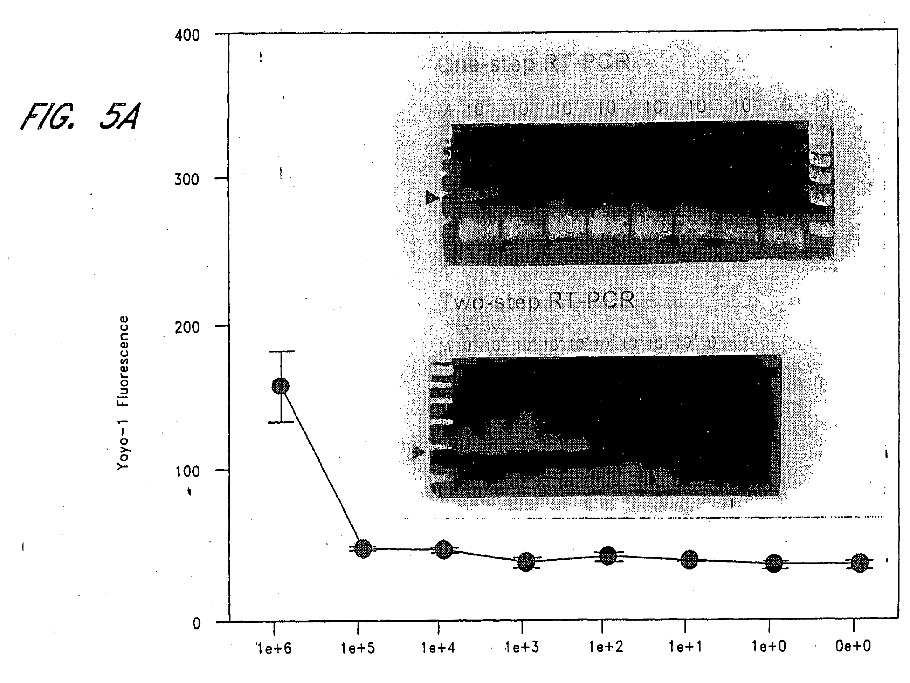

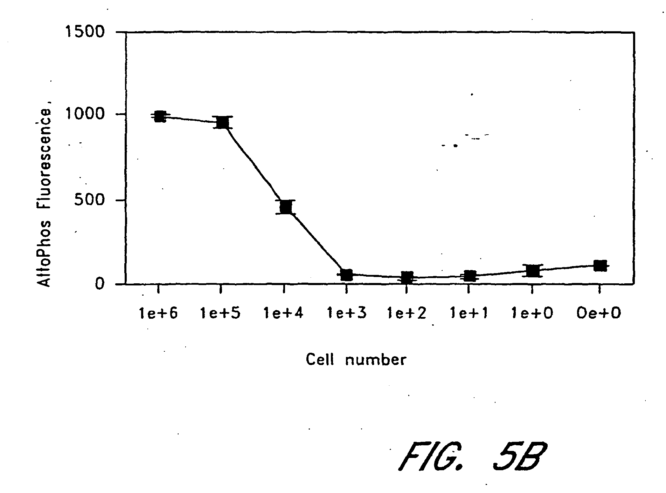

Direct RT-PCR experiments were conducted at different dilutions of cell suspension. Various numbers of K562 cells were applied to the origonuclootide-immobilized PCR microplates for hybridization. The resultant hybridized mRNA was used for either measurement of Yoyo-1, or one-step RT-PCR using rTth polymerase. That is, in Figs. 5A and 5B, various amounts 10-6x10⁶) of K562 cells were suspended in lysis, and were applied to the oligonucleotide-immobilized PCR microplates for hybridization. In Fig. 5A, the amounts of hybridized mRNA were determined by YOYO^{™}.1 fluorescence, as described above. In parallel experiments, captured mRNA was immediately subjected to one-step RT-PCR using rTth polymerase, as described above (Inset, upper). In Fig. 5B, in another series of experiments, cDNA was synthesized from captured mRNA using MMLV reverse transcriptase and immobilized ofigo(dT) as a primer in the presence of biotin dUTP, followed by quantitation of cDNA synthesis, as described above. In parallel experiments, cDNA was synthesized on the oligonucleotide-immobilized PCR microplates by replacing biotin-dUTP with unlabeled dTTP, and PCR was conducted with rTth polymerase, as shown above (Inset, lower). The PCR products were separated by 2.0% agarose gel electrophoresis followed by staining with ethidium bromide. M indicates a 100 bp ladder. Each data point was the mean±S.D. from triplicate determinations.

As shown in Fig. 5B, significant ATTOPHOS™ signals were obtained even from as few as 10⁴ cells, suggesting 100-fold more sensitivity than YOYO^{™}-1. More advantageously, when PCR was conducted from synthesized cDNA on the oligonucleotide-immobilized PCR microplates, PCR band was detected from as few as 10 cells (Fig. 5A, inset bottom).

In view of the foregoing, RT-PCR from synthesized cDNA on the oligonucleotide-immobilized PCR microplates (Two-step RT-PCR, Fig. 5A, lower inset) is approximately 100,000-fold more sensitive than conventional one-step RT-PCR, and bcr-abl transcript was detected from cell lysates containing only 10 cells (Fig. 5A, top inset). This is surprising because two-step RT-PCR required inefficient solid phase reverse transcription reaction, whereas one-step RT-PCR was conducted in more efficient liquid phase reaction by first dissociating mRNA from the oligonucleotide-immobilized PCR microplates. Since rTth was used for both experiments, the difference was not due to the enzyme. Because more primer dimers were formed in one-step PCR than two-step RT-PCR (Fig. 5A, top and lower insets: the clear band on each lane in the top inset indicate primer dimers), it is believed that primers are used for dimer formation during reverse transcription. In two-step RT-PCR, these primer dimers can be removed when the reaction mixture was switched from cDNA synthesis to PCR, whereas primer dimers formed during the initial reverse transcription phase exist throughout PCR.

### Experiment 7: Intra- and Inter-Assay of Oligonucleotide Immobilization, Hybridization, and cDNA Synthesis

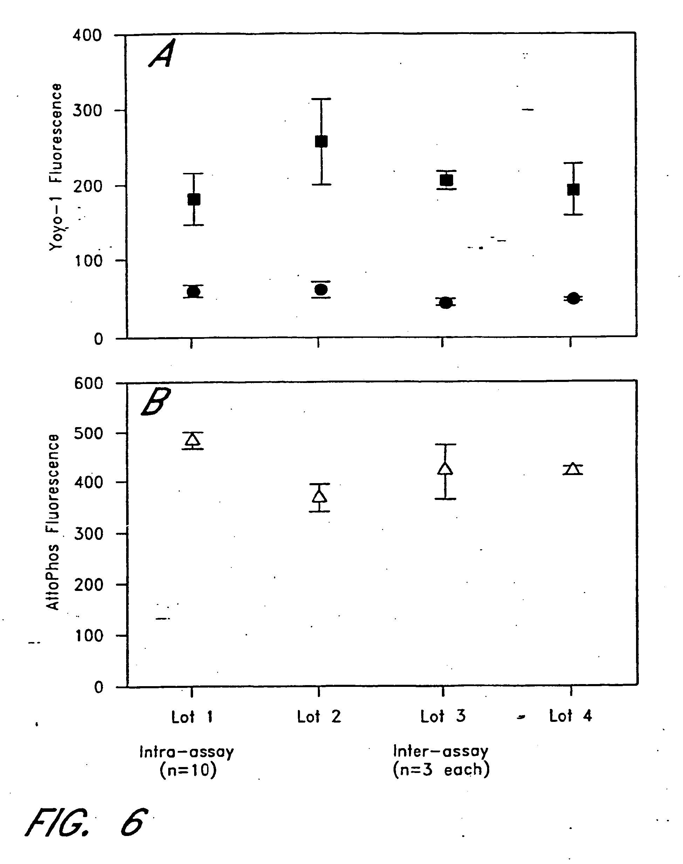

In order to conduct quantitative analysis on the oligonucleotide-immobilzed PCR microplates, well-to-well variation is a critical issue. One hundred pmol of oligonucleotides were applied to the oligonucleotide-immobilized PCR microplates for immobilization followed by VOYOTM-1 fluorescence determination in a fluorescent plate reader, as described above (Fig. 6A●). One hundred ng of rabbit globin mRNA was applied to each well for hybridization, followed by YOYO™-1 fluorescence determination in a fluorescent plate reader, as described above (Fig. 6A■). One hundred ng of rabbit globin mRNA was applied to each well for hybridization, followed by cONA synthesis in the presence of biotin-dUTP. ATTOPHOS™ fluorescence was then determined in a fluorescent plate reader, as described above (Fig. 6BΔ). Each data point was the mean±S.D. from 10 (intra-assay) to 3 (Inter-assay) separate determinations.

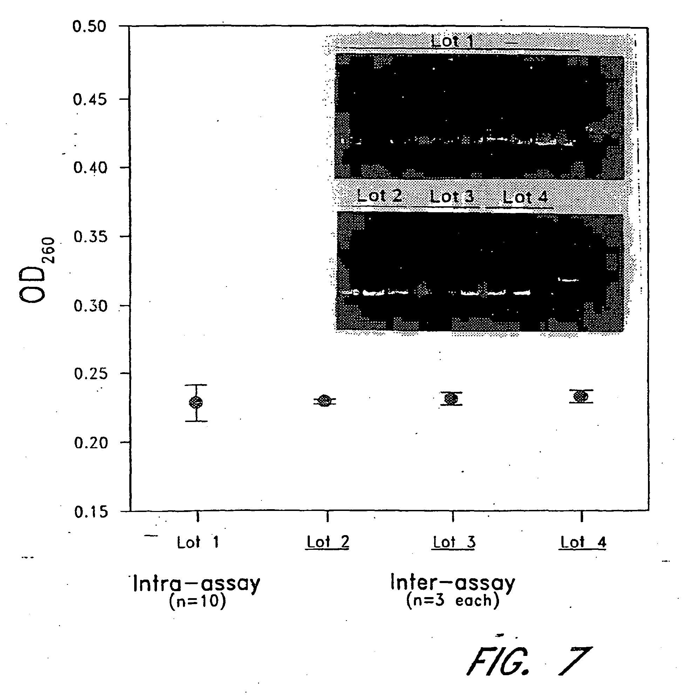

As shown in Figs. 6A and 6B, variation of the amounts of immobilized oligonucleotides (Fig. 6A●), hybridized rabbit globin mRNA (Fig. 6A■), and synthesized cDNA from captured rabbit globin mRNA (Fig. 6BΔ) were all less than 10-15% within a single microplate (Intra-assay) or multiple lots of microplates (Inter-assay). More importantly, the variation of the amount of PCR products in these intra- and inter-assays were also within 10% (Fig. 7). In Fig. 7, one hundred ng of rabbit globin mRNA was applied to each well for hybridization, followed by cDNA synthesis in the presence of unlabeled dTTP. PCR was then conducted with rabbit globin specific primers and Taq polymerase, as described in the Methods. The PCR products were separated by 2.0% agarose gel electrophoresis followed by staining with ethidium bromide. Right lanes indicate a 100 bp ladder. The amounts of PCR products were determined by measuring OD₂₆₀ (●). Each data point was the mean±S.D. from 10 (Intra-assay) to 3 (Inter-assay) separate determinations.

In view of the foregoing, less variation among wells and plates, excellent stability, and availability of various quality control protocols (e.g., Figs. 6A, 6B, 7) make this technology very competitive.

### Experiment 8: Stability of oligonucleotide-immobilized PCR microplates

The oligonucleotide-immobilized PCR microplates were stored at room temperature (●), 55°C (■) or 72°C (Δ) for 2, 8 or 15 days. One hundred ng of rabbit globin mRNA was then applied to each well for hybridization, followed by cDNA synthesis in the presence of biotin-dUTP. ATTOPHOS^{™} fluorescence was then determined in a fluorescent plate reader, as described above. Each data point was the mean±S.D. from triplicate determinations.

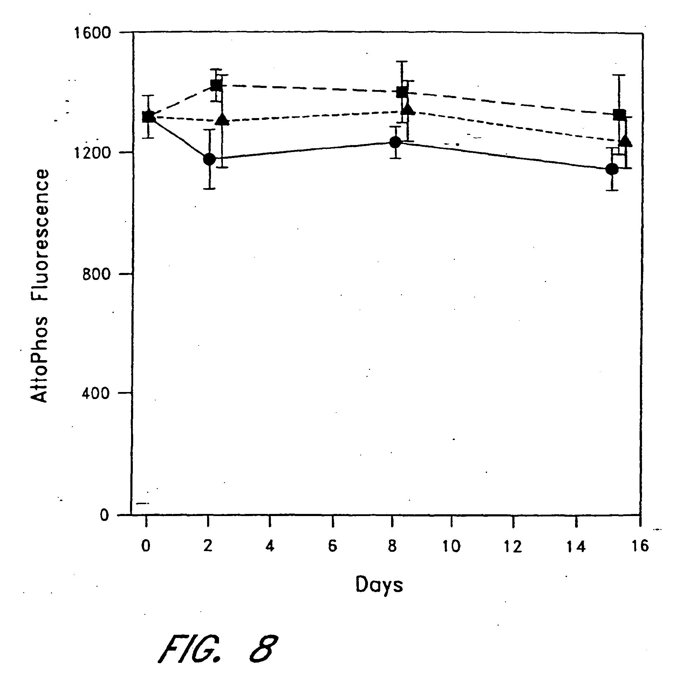

As shown in Fig. 8, quantities of cDNA synthesis did not show any significant decreases even after storage at 72°C for 15 days.

### Experiment 9: Multiple PCRs from cDNA Synthesized on oligonucleotide-immobilized PCR microplates

K562 cells (10⁴-10⁶) were suspended in Lysis buffer and were applied to the oligonucleotide-immobilized PCR microplates for hybridization. The captured mRNA was converted to cDNA with MMLV reverse transcriptase as described above. As controls, some wells were treated identically but without MMLV reverse transcriptase. Then bcr-abl transcript was amplified by PCR with Taq polymerase, as described above (1st bcr-abl). After PCR, each well was washed with boiling DEPC water five times, and PCR was repeated with the same primer set (2nd bcr-abl). PCR was then repeated a third time with primer pair from G3PDH (3rd G3PDH). The PCR products were separated by 2.0% agarose gel electrophoresis, followed by staining with ethidium bromide. As a result, the agarose gel electrophoresis indicates that PCR products of bcr-abl and G3PDH transcripts were not amplified from the wells of negative reverse transcription, indicating no "false" PCR products from contaminating genomic cDNA in the plates. More interestingly, the agarose gel electrophoresis confirms that bcr-abl and G3PDH transcripts were reamplified plural times from immobilized cDNAs from wells.

### Experiment 10: Collection of Cytosolic mRNA Fraction to oligonucleotide-immobilized PCR microplates from Various Cells by Glass Fiber Filter

Various human cultured cell lines were used in this experiment: K562 leukemic, U937 leukemic, CaRl colon cancer, HepGll hepatoma, Katolli stomach cancer, and CRL 5800 lung adenocarcinoma (American Type Culture Collection, Rockville, MD). A 96-well filter plate with a single layer was made of glass fiber (Cambride Technology grade 934AH, Brandel, Gaithersburg, MD) in order to trap cells thereon. In preliminary experiments, the maximum capacity of cells trapped on the single layer of glass fiber filter membranes in each well of the 96-well filter plate was determined. Various numbers of cells (10² to 5x10⁶) were applied to the filter plate assembled on top of a regular 96-well microplate, and centrifuged at 500 xg for 10 min. The number of cells in the passed-through fraction collected in the well of the lower plates was measured with a hemocytometer. As a result, the maximum capacity of cells per well was approximately 2x10⁶, 2x10⁶, 10⁶, 5x10⁵, 5x10⁵ and 3x10⁵ for K562, U937, CaRl, HepGll, Katolll, and CRL 5800 cells, respectively, without any leakage of cells from glass fiber membranes.

In the next series of experiments, 10⁵ cells were applied to the filter plate, and cells were trapped onto the membrane by vacuum aspiration. The membranes were washed twice with PBS, and placed on top of the oligo(dT)-immobilized polypropylene/polyolefine microplate (GENEPLATE^{®}-PP, AGCT) which would subsequently be used as a oligonucleotide-immobilized PCR microplate. Fifty µL of lysis buffer (10 mM Tris, pH 8.0, 1 mM EDTA, 0.5 M NaCl, 0.5% NP-40 detergent, and 20 mM vanadyl ribonucleoside complex (VRC, Gibco-BRL, Geithersburg, MD)) was added to each well, and was immediately centrifuged at 500 xg for 10 min to recover cytosolic RNA fraction into the oligonucleotide-immobilized PCR microplate. The lysis buffer allowed hybridization between oligo(dT) and poly(A) sequences of mRNA in the presence of RNase inhibitor VRC. After hybridization at room temperature for 1 hr, the oligonucleotide-immobilized PCR microplate was washed twice with a wash buffer (10 mM Tris, pH 8.0, 1 mM EDTA, and 0.5 M NaCl). At this stage, total mRNA was captured in each well of the oligonucleotide-immobilized PCR microplate for analysis. Because of the heat-stable characteristics of the oligonucleotide-immobilized PCR microplate, the oligonucleotide-immobilized PCR microplates were directly subjected to PCR without transfer of mRNA to PCR vessels.

### Experiment 11: Measurement of mRNA and PCR amplification of β-actin from various cultured cells

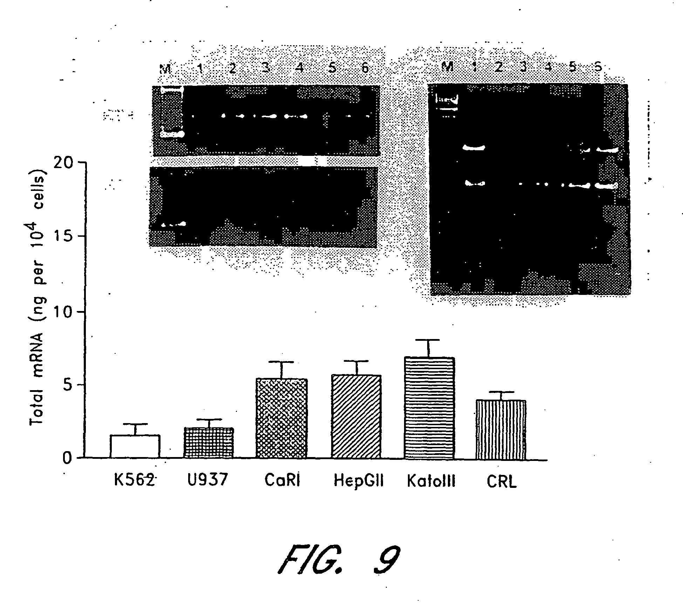

Subsequent to Experiment 10, the first analysis was to amplify housekeeping genes from captured mRNA. The cDNA was synthesized by adding RT-buffer (50 mM Tris, pH 8.3, containing 75 mM KCl, 3 mM MgCl₂, and 10 mM DTT; 10 mM of each dNTP; and 100 U of MMLV reverse transcriptase (Gibco-BRL), and incubated at 37°C for 1 hour. PCR was then conducted on the same plate, by replacing RT-buffer with PCR-buffer (10X PCR buffer, 1.5 mM MgCl₂, 100 µM of dNTPs, 1.5 units of Taq DNA polymerase (Perkin Elmer, Fostercity, CA), 0.5 µM each of upstream sense primer and downstream antisense primer of human β-actin (Clontech, Palo Alto, CA) in a final volume of 20 µL. PCR was conducted in a thermal cycler (MJ Research, PTC-100, Watertown, MA) with 35 cycles of 45 seconds at 94°C, 45 seconds at 60°C and 2 minutes at 72°C. PCR products were analyzed in a 1.0% agarose gel electrophoresis with 0.5 µg/mL ethidium bromide. As shown in Fig. 9 (left insets: lane M, 100 bp DNA ladder; lane 1 K562; lane 2, U937; lane 3, CaRl; lane 4, HepGll; lane 5, Katolll; lane 6, CRL5800), β-actin gene was successfully amplified from various cultured cells. Since intron sequences exist between sense and antisense primers, the size of β-actin PCR products were equal to that of intron-free mRNA. Furthermore, when starting PCR without cDNA synthesis, the β-actin gene was not amplified, suggesting that PCR was mRNA-specific, and not derived from contaminated DNA.

In parallel experiments, the amounts of captured mRNA on the oligonucleotide-immobilized PCR microplate were quantitated by the method published previously from our laboratory (Tominaga K, et al., "Colorimetric ELISA measurement of specific mRNA on immobilized-oligonucleatide-coated microtiter plates by reverse transcription with biotinylated mononucleotides", Clin Chem 1996:1750-1757, 1996) with minor modifications. In brief, the first strand cDNA was synthesized on the microplate by adding RT-buffer containing 250 µM biotin-dUTP instead of 10 mM of dTTP, and incubated at 37°C for 1 hour. Each well was washed three times with wash buffer, and 50 µL of wash buffer containing 1:1000 dilution of streptavidin-alkaline phosphate conjugates (Clontech, Palo Alto, CA) were added to each well. Each well was incubated at room temperature for 30 min, and then washed three times with wash buffer. Finally 100 µL of AttoPhos (JBL Scientific, San Luis Obispo, CA) was added to each well and incubated at room temperature for 15 min. Fluorescence was determined in a CytoFluor 2300 (Millipore, Bedford, MA) at 430 nm excitation and 560 nm emission. In order to quantitate the amount of mRNA from fluorescence intensity, rabbit globin mRNA was used as a control as previously described (Tominaga K, et al., Clin Chem 1996:1750-1757, 1996). As shown in Fig. 9, captured mRNA from 10⁴ cells was approximately 5 ng from 5 different cell lines.

In order to further analyze the potential degradation of mRNA during hybridization, cytosolic fraction was collected after hybridization, and treated with two rounds of phenolichloroformfisoamyl alcohol extraction followed by ethanol precipitation. RNA was then analyzed by agarose gel electrophoresis. As shown in Fig. 9 (right inset:
Lane M, λ Hind III; lane 1, K562; lane 2, U937; lane 3, CaRl; lane 4, HepGll; lane 5, Katolll; lane 6, CRL5800), 18s and 28s rRNA bands were clearly present in all cells even after 1 hour incubation at room temperature, suggesting that simple cytosolic fraction in the presence of VRC was essentially free from RNase activity.

In conclusion, complete RT-PCR from starting cell suspension can be conducted using just two plates; the glass fiber filter plate and the oiigo(dT)-immobilized polypropylene plate. Furthermore, the 96-well format allows researchers to conduct RT-PCR in high throughput fashion with potential full automation. In this experiment, PCR products were analyzed by agarose gel electrophoresis, however, PCR products may be quantitated continuously by TaqMan system (Morris T, et al., J Clin Microbiol 34:p2933-6, 1996). This experiment proved that this system is a useful tool for high throughput RT-PCR.

## Claims

1. A method of reverse transcription-polymerase chain rection (RT-PCR), comprising the steps of:
(a) preparing cell lysate of a target cell;
(b) transferring the cell lysate to an oligonucleotide-immobilized microplate having wells to which oligonucleotides are securely immobilized, said microplate having heat-stability for thermal cycles of PCR and being made of polypropylene, said oligonucleotides having nucleic acid sequences specifically complementary to mRNA of interest present in the cell lysate, or said oligonucleotides being oligo (dT);
(c) capturing mRNA by the oligonucleotides of the microplate;
(d) conducting solid-phase RT-PCR on the same microplate, using an appropriate buffer wherein,
(i) captured mRNA is reverse transcribed to cDNA;
(ii) reactants are removed by aspiration; and
(iii) PCR is conducted by using heat stable DNA polymerase on the microplate to which the cDNA has been immobilized; and
(e) detecting PCR products of interest.

2. A method of RT-PCR according to claim 1, wherein, in the detection step, the PCR products are quantitated fluorometrically by using nucleic acid stain or enzymatically by producing fluorescence or chemiluminuscence.

3. A method of RT-PCR according to claim 1 or 2, wherein, in the transfer step, crude cell lysate, without purification of RNA or mRNA, is transferred to the microplate.

4. A method of RT-PCR according to any one of claims 1 to 3, wherein, in the cell lysate preparation step, cell lysate is prepared using a lysis buffer comprising a mild detergent for destructing cell membranes but maintaining nuclei to be intact and a reagent for inhibiting RNase activity or inactivating RNase, said lysis buffer having a pH and salt concentration for hybridization.

5. A method of RT-PCR according to any one of claims 1 to 4, further comprising the steps of :
after detecting the PCR products, washing the microplate wherein the cDNA synthesized on the microplate remains immobilized;
conducting PCR with appropriate primers, said primers being the same as or different from those used in the RT-PCR step; and
measuring PCR products of interest.

6. A method of RT-PCR according to claim 5, wherein the washing step, the PCR step, and the measuring step are repeated.

## Patentansprüche

1. Verfahren einer reversen Transkriptions-Polymerasenkettenreaktion (RT-PCR), umfassend die Schritte:
(a) Herstellen von Zelllysat einer Zielzelle;
(b) Transferieren des Zelllysats auf eine Mikrotiterplatte mit immoblisierten Oligonucleotiden, welche Vertiefungen hat, an die die Oligonucleotide fest immobilisiert sind, wobei die Mikrotiterplatte Hitzestabilität gegenüber thermischen PCR-Zyklen aufweist und aus Polypropylen hergestellt ist, wobei die Oligonucleotide Nucleinsäuresequenzen haben, welche spezifisch komplementär zur mRNA von Interesse sind, welche im Zelllysat vorliegt, oder wobei die Oligonucleotide Oligo(dT) sind;
(c) Einfangen von mRNA durch die Oligonucleotide der Mikrotiterplatte;
(d) Durchführen von Festphasen-RT-PCR auf derselben Mikrotiterplatte unter Verwendung eines geeigneten Puffers, wobei
(i) die eingefangene mRNA revers zur cDNA transkribiert wird;
(ii) Reaktanten durch Aspiration entfernt werden; und
(iii) die PCR unter Verwendung von hitzestabiler DNA-Polymerase auf der Mikrotiterplatte, an welche die cDNA immobilisiert wurde, durchgeführt wird; und
(e) Nachweis von PCR-Produkten von Interesse.

2. RT-PCR-Verfahren nach Anspruch 1, wobei im Nachweisschritt die PCR-Produkte fluorimetrisch unter Verwendung eines Nucleinsäure-Färbemittels oder enzymatisch durch die Erzeugung von Fluoreszenz oder Chemilumineszenz quantifiziert werden.

3. RT-PCR-Verfahren nach Anspruch 1 oder 2, wobei im Transferschritt unbehandeltes Zelllysat ohne Reinigung von RNA oder mRNA auf die Mikrotiterplatte transferiert wird.

4. RT-PCR-Verfahren nach einem der Ansprüche 1 bis 3, wobei im Zelllysat-Herstellungsschritt das Zelllysat unter Verwendung eines Lysepuffers hergestellt wird, der ein mildes Detergens zur Zerstörung von Zellmembranen, wobei die Zellkerne intakt bleiben, und ein Reagens zur Hemmung von RNase-Aktivität oder zur Inaktivierung von RNase umfasst, wobei der Lysepuffer einen pH-Wert und eine Salzkonzentration für die Hybridisierung besitzt.

5. RT-PCR-Verfahren nach einem der Ansprüche 1 bis 4, zusätzlich umfassend die Schritte:
- nach dem Nachweis der PCR-Produkte Waschen der Mikrotiterplatte, wobei die synthesierte cDNA auf der Mikrotiterplatte immobilisiert bleibt,
- Durchführen einer PCR mit geeigneten Primern, wobei die Primer gleich oder unterschiedlich sind im Vergleich zu jenen, welche im RT-PCR-Schritt verwendet wurden; und
- Messen der PCR-Produkte von Interesse.

6. RT-PCR-Verfahren nach Anspruch 5, wobei der Waschschritt, der PCR-Schritt und der Messschritt wiederholt werden.

## Revendications

1. Méthode de réaction d'amplification en chaîne par polymérase après transcription inverse (RT-PCR), comprenant les étapes consistant à :
(a) préparer un lysat cellulaire d'une cellule cible ;
(b) transférer le lysat cellulaire sur une microplaque à oligonucléotides immobilisés ayant des puits auxquels des oligonucléotides sont solidement immobilisés, ladite microplaque étant thermostable lors des cycles thermiques de PCR et étant faite en polypropylène, lesdits oligonucléotides ayant des séquences d'acide nucléique spécifiquement complémentaires de l'ARNm d'intérêt présent dans le lysat cellulaire, ou lesdits oligonucléotides étant des oligos (dT) ;
(c) capturer l'ARNm grâce aux oligonucléotides de la microplaque ;
(d) réaliser une RT-PCR en phase solide sur la même microplaque, en utilisant un tampon approprié, dans laquelle
(i) l'ARNm capturé est rétrotranscrit en ADNc ;
(ii) les réactifs sont retirés par aspiration ; et
(iii) la PCR est réalisée en utilisant une ADN polymérase thermostable sur la microplaque sur laquelle l'ADNc a été immobilisé ; et
(e) détecter les produits PCR d'intérêt.

2. Méthode de RT-PCR selon la revendication 1, dans laquelle, dans l'étape de détection, les produits PCR sont quantifiés par fluorométrie en utilisant un colorant d'acide nucléique ou enzymatiquement par production d'une fluorescence ou d'une chimioluminescence.

3. Méthode de RT-PCR selon la revendication 1 ou 2, dans laquelle, lors de l'étape de transfert, le lysat cellulaire brut, sans purification de l'ARN ou de l'ARNm, est transféré sur la microplaque.

4. Méthode de RT-PCR selon l'une quelconque des revendications 1 à 3, dans laquelle, lors de l'étape de préparation du lysat cellulaire, le lysat cellulaire est préparé en utilisant un tampon de lyse comprenant un détergent doux pour détruire les membranes cellulaires tout en conservant les noyaux intacts et un réactif pour inhiber l'activité ARNase ou inactiver l'ARNase, ledit tampon de lyse ayant un pH et une concentration en sels pour l'hybridation.

5. Méthode de RT-PCR selon l'une quelconque des revendications 1 à 4, comprenant en outre les étapes consistant à :
après la détection des produits PCR, laver la microplaque dans laquelle l'ADNc synthétisé sur la microplaque reste immobilisé ;
réaliser une PCR avec des amorces appropriées, lesdites amorces étant les mêmes ou étant différentes de celles utilisées lors de l'étape de RT-PCR ; et
mesurer les produits PCR d'intérêt.

6. Méthode de RT-PCR selon la revendication 5, dans laquelle l'étape de lavage, l'étape de PCR, et l'étape de mesure sont répétées.
